# EUROPÄISCHE PATENTSCHRIFT

(11) **EP 2 155 063 B1**
(45) Veröffentlichungstag und Bekanntmachung des Hinweises auf die Patenterteilung: **01.05.2013**
(21) Anmeldenummer: 08759470.1
(22) Anmeldetag: 08.05.2008
(51) Int. Cl.: A61B 5/151

(54) **VERFAHREN ZUR MAGAZINIERUNG VON STECHELEMENTEN**
METHOD FOR MAGAZINING PUNCTURING ELEMENTS
PROCÉDÉ D'ENTREPOSAGE D'ÉLÉMENTS DE PERFORATION

(30) Priorität: 15.05.2007 EP 07108252
(43) Veröffentlichungstag der Anmeldung: 24.02.2010
(73) Patentinhaber: F. Hoffmann-La Roche AG, 4070 Basel (CH); Roche Diagnostics GmbH, 68305 Mannheim (DE)
(72) Erfinder: ZIMMER, Volker, 54497 Morbach (DE); DECK, Frank, 67150 Niederkirchen (DE); HILLER, Bernd, 68623 Lampertheim (DE); KONYA, Ahmet, 67165 Waldsee (DE)
(74) Vertreter: Pfiz, Thomas
(86) Internationale Anmeldenummer: PCT/EP2008/055689
(87) Internationale Veröffentlichungsnummer: WO 2008/138857

(56) Entgegenhaltungen:
- EP-A- 1 466 558
- EP-A- 1 593 434
- EP-A- 1 714 613
- WO-A-2004/060174
- WO-A-2005/107596
- WO-A2-2008/043565
- DE-B1- 2 803 345
- GB-A- 1 073 596
- US-A1- 2003 059 132
- US-A1- 2003 083 685

## Beschreibung

Die Erfindung betrifft ein Verfahren zur Magazinierung von Lanzetten und ggf. Testelementen insbesondere für Blutglucoseuntersuchungen bei dem eine Vielzahl von mit einer Lanzettenspitze zum Einstechen in die Haut versehenen disposiblen Lanzetten auf einem auf einer Spule aufgewickelten oder einem aufwickelbaren Trägerband zum Gewinnen einer Körperflüssigkeitsprobe bereitgestellt werden, wobei die Lanzetten in Bandquerrichtung des Trägerbandes ausgerichtet sind.

Ein Verfahren dieser Art ist aus der US 2003/083685 A1 bekannt. Die Lanzetten sind dort über eine Folie miteinander verbunden, die zugleich die Funktion eines Trägerbandes und einer Sterilverpackung erfüllen soll.

In der WO 2004/060174 A wird eine Verpackung für einzelne Lanzetten offenbart, die aber nicht geeignet ist, um auf ein Trägerband aufgebracht zu werden, weil sich diese Verpackung beim Öffnen in Längsrichtung der jeweiligen Lanzette aufrollt.

In der EP-A-1 466 558 werden sterile Lanzettenpackungen offenbart, die zunächst als Gurt hergestellt und anschließend vereinzelt werden.

Die Gemäß Artikel 54 (3) EPÜ als Stand der Technik bei der Neuheitsprüfung zu berücksichtigende WO 2008/043565 A2 offenbart nur Lanzetten, die in Bandlängsrichtung eines Trägerbandes aufgebracht sind.

Aus der WO-A 2005/107596 ist eine Anordnung von Lanzetten auf einem Trägerband bekannt, wobei sich die einzelnen Lanzetten durch Bandtransport sukzessive zum Einsatz bringen und auch wieder zu entsorgen lassen. Die Lanzetten sollen im Zuge der Fertigung durch eine direkte Verbindung auf dem Band integriert werden. Daneben ist auch eine zusätzliche Bereitstellung eines Testmediums auf dem Band offenbart. Damit kann dem Benutzer die Handhabung bei der Durchführung von Selbsttests insbesondere für die Blutzuckerüberwachung erleichtert werden.

Ausgehend hiervon liegt der Erfindung die Aufgabe zugrunde, die im Stand der Technik bekannten Fertigungsverfahren und Verfahrenserzeugnisse weiter zu verbessern und so zu gestalten, dass eine günstige Massenproduktion bei hoher Produktqualität möglich ist.

Zur Lösung dieser Aufgabe wird die im Patentanspruch 1 angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, die disposiblen Elemente gesondert auf einem ersten Träger zu verpacken und dann etikettenartig auf einem zweiten Träger zu applizieren. Dementsprechend wird erfindungsgemäß vorgeschlagen, dass die Lanzetten in jeweils einer Packung angeordnet und dabei zumindest im Bereich ihrer in die Haut eindringenden Spitze vor dem Aufbringen auf das Trägerband sterilisiert und steril dicht eingeschlossen werden, und dass anschließend die so gebildeten Lanzetten-Packungen vereinzelt werden und einzeln auf das Trägerband aufgebracht und darauf fixiert werden. Auf diese Weise ergeben sich für die Magazinierung steril zu haltender Stechelemente besondere Vorteile. Die Vorverpackung erlaubt einen Sterilschutz auch in einer Fertigungsumgebung und vereinfacht zugleich die maschinelle Handhabung bei der Verbindung mit dem Trägerband. Zudem ergibt sich durch die Verpackung auch eine erhöhte Lagerstabilität.

Um eine flache, aufrollbare Struktur zu schaffen, ist es vorteilhaft, wenn die Packungen aus einer Bodenfolie und einer damit verbundenen Deckfolie gebildet werden. Hierbei können die Bodenfolie und die Deckfolie unter Einschluss der Lanzetten miteinander in Kontakt gebracht und verbunden werden.

Zusätzliche Bauteile lassen sich dadurch vermeiden, dass die Bodenfolie und die Deckfolie über eine Schmelzkleberverbindung miteinander verbunden werden, wobei eine vorzugsweise auf der Bodenfolie befindliche Schmelzkleberschicht durch einen Heißstempel aktiviert wird.

Herstellungsvorteile ergeben sich dadurch, dass eine Vielzahl von Lanzetten-Packungen als Packungsband zusammenhängend gefertigt und vor dem Aufbringen auf das Trägerband vereinzelt wird. Eine weitere fertigungstechnische Vereinfachung sieht vor, dass das Packungsband durch Zusammenführen und Verbinden eines Bodenfolienstreifens und eines Deckfolienstreifens gebildet wird. Alternativ ist es auch möglich, dass das Packungsband durch Umklappen eines Folienstreifens und Bildung von Lanzettentaschen in dem gefalteten Folienstreifen gefertigt wird.

Eine Sterilverpackung lässt sich dadurch erreichen, dass die Packungen durch einen Heißschnitt zugleich zugeschnitten und unter Einschluss der Lanzetten dicht umrandet werden.

Um eine gerätetechnische Handhabung zu vereinfachen, ist es vorteilhaft, wenn die Lanzetten vorzugsweise an einem proximalen Endabschnitt fest mit der Packung und vorzugsweise mit der Bodenfolie verbunden werden.

Eine besonders bevorzugte Ausgestaltung sieht vor, dass die Lanzetten zumindest im Bereich ihrer Spitze in der Packung gegen Beschädigung geschützt und/oder steril gehalten sind.

Vorteilhafterweise werden die Lanzettenspitzen in einer durch die Packung gebildeten Tasche angeordnet. Denkbar ist es auch, dass die Packungen durch eine Bodenfolie und eine darauf aufgebrachte und zumindest die Lanzettenspitze umschließende, insbesondere aus Silikon oder TPE bestehende biokompatible Verschlussmasse gebildet werden. In jedem Fall sollte die Tasche bzw. der Verschlussblock leicht penetrierbar sein, um die Lanzettenspitze für den Gebrauch freizugeben.

Eine weitere vorteilhafte Ausgestaltung besteht darin, dass zumindest die Spitzen der Lanzetten in den Lanzetten-Packungen vor dem Aufbringen auf das Trägerband vorzugsweise durch hochenergetische Bestrahlung sterilisiert werden. Damit lässt sich sowohl ein gezielter Sterilschutz einer hohen Stückzahl als auch eine nachfolgende Handhabung der Lanzetten-Packungen außerhalb eines Sterilbereichs realisieren.

Die Applikation auf das Trägerband lässt sich dadurch einfach bewerkstelligen, dass die Lanzetten-Packungen jeweils an einer von der Lanzette abgewandten Verbindungsfläche auf dem Trägerband fixiert werden. Besonders bevorzugt werden die Lanzetten-Packungen in Form von Etiketten oder Aufklebern auf das Trägerband aufgebracht. Hierbei ist es günstig, wenn die Lanzetten-Packungen mit einem Klebebandstück zum Aufkleben auf das Trägerband versehen werden.

Grundsätzlich ist es auch möglich, dass die Lanzetten-Packungen durch mechanische Verbindungsmittel oder durch Verschweißen (z.B. mittels Ultraschall oder Laser) auf dem Trägerband gehalten werden.

Eine besonders bevorzugte Variante der Erfindung sieht vor, dass auf dem Trägerband vorzugsweise alternierend zu den Lanzetten-Packungen mit der Köperflüssigkeitsprobe beaufschlagbare Testelemente aufgebracht werden. Dementsprechend wird vorgeschlagen, dass die Lanzetten und die Testelemente gesondert als Packungseinheiten vorgefertigt werden, und dass anschließend in einem einheitlichen Bestückungsprozess die Packungseinheiten auf das Trägerband aufgebracht und darauf fixiert werden. Auf diese Weise lassen sich völlig unterschiedliche disposible Einheiten beim Aufbringen auf das Band einheitlich handhaben. Dabei kann durch die gleichartige, aber separat durchgeführte Vorverpackung unterschiedlichen Anforderungen hinsichtlich Sterilschutz der Lanzetten und Schutz der Testchemie in besonderem Maß Rechnung getragen werden.

Eine weitere Fertigungsvereinfachung ergibt sich dadurch, dass die flachen Lanzetten- und Testelement-Packungseinheiten eine im Wesentlichen übereinstimmende, vorzugsweise der Breite des Trägerbands entsprechende Breite aufweisen.

Weitere Gebrauchsvorteile lassen sich auch dadurch erzielen, dass das Trägerband in einer Kassette angeordnet wird, so dass es von einer Abwickelspule abziehbar und auf eine Aufwickelspule aufspulbar ist.

Im Folgenden wird die Erfindung anhand der in der Zeichnung schematisch dargestellten Ausführungsbeispiele näher erläutert. Es zeigen
- Fig. 1: ein Lanzettenband in einer abgebrochenen perspektivischen Darstellung;
- Fig. 2: eine Ausschnittsvergrößerung des Lanzettenbands nach Fig. 1;
- Fig. 3: den Schichtaufbau des Lanzettenbands in einer Explosionsdarstellung;
- Fig. 4 und 5: einen Schnitt durch das Lanzettenband in Bandquerrichtung und Bandlängsrichtung;
- Fig. 6 und 7: eine schaubildliche Darstellung verschiedener Schritte zur Fertigung von Lanzetten-Packungen;
- Fig. 8: einen Stempel zum Heißsiegeln der Lanzetten-Packungen in der Draufsicht;
- Fig. 9: einen Schnitt entlang der Linie 9 - 9 der Fig. 8;
- Fig. 10: eine Bandkassette mit einem mit Lanzetten und Testelementen bestückten Testband in teilweise aufgebrochener perspektivischer Darstellung;
- Fig. 11 und 12: die Ingebrauchnahme einer Lanzette in perspektivischer und geschnittener Ansicht.

Die in der Zeichnung dargestellten Lanzettenvorratsbänder 10 ermöglichen die Magazinierung bzw. Bereitstellung einer Vielzahl von Lanzetten 12 als Einmalartikel für Blutglucosebestimmungen oder andere Untersuchungen, bei denen durch einen Hauteinstich eine Körperflüssigkeitsprobe für diagnostische Zwecke gewonnen werden soll. Daneben sind auch Kombinationen von Lanzetten und diagnostischen Testelementen auf einem gemeinsamen Vorratsband möglich. In beiden Fällen werden vorgefertigte Packungs- oder Trägereinheiten bereitgestellt, die auf einem Trägerband in Form eines Bandwickels magaziniert werden.

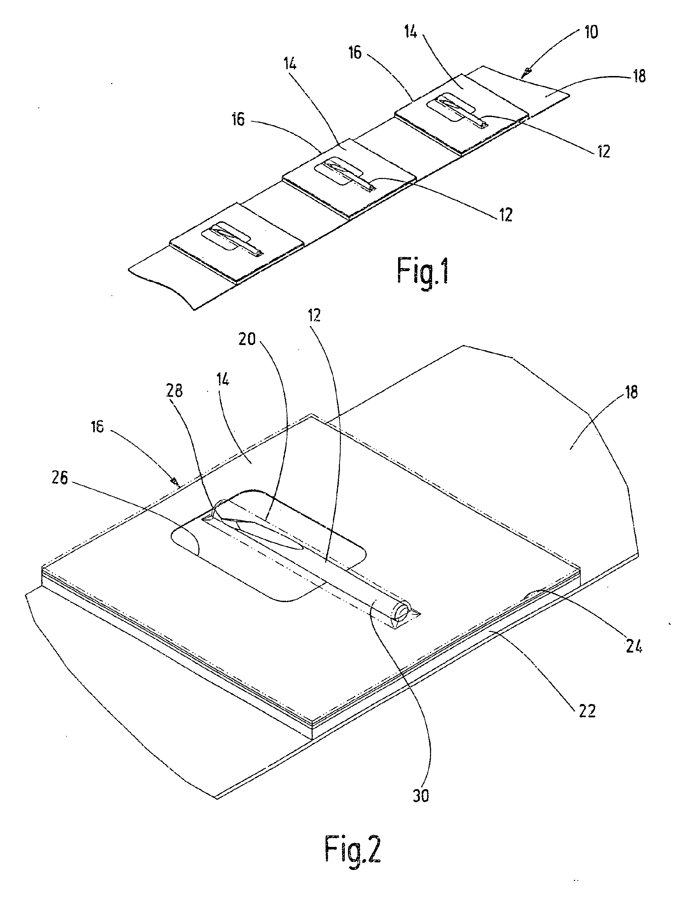

Wie in Fig. 1 gezeigt, sind die Lanzetten 12 in jeweils einer zugeordneten Packung 14 angeordnet, wobei die so gebildeten Verpackungseinheiten bzw. Lanzetten-Packungen 16 im Abstand voneinander nach Art von Etiketten auf dem Trägerband 18 fixiert sind. Aufgrund der flexiblen und flachen Lanzetten-Packungen 16 ergibt sich so ein aufrollbares Bandmagazin, das in ein nicht gezeigtes Handgerät zur automatischen Handhabung einsetzbar ist.

In der Vergrößerung nach Fig. 2 ist zu ersehen, dass die jeweilige Lanzette 12 in einer durch die Packung 14 gebildeten Tasche 20 geschützt ist. Die Tasche 20 wird durch einen Folienverbund aus einer Bodenfolie 22 und einer Deckfolie 24 gebildet. Ein erweiterter Taschenbereich 26 nimmt die Lanzettenspitze 28 freiliegend auf, während der proximale Schaftabschnitt 30 der Lanzette 12 eng eingeschlossen wird. Ein maschineller Umgang auch mit sehr kleinen Nadelelementen wird somit erleichtert, ohne dass eine Beschädigung der sehr empfindlichen Spitze sowie eine Beeinträchtigung von deren Sterilität befürchtet werden muss. In der gezeigten Ausführung ist eine in Bandquerrichtung ausgerichtete Rundlanzette vorgesehen. Denkbar sind auch andere Orientierungen und Ausformungen, beispielsweise in Form eines mit einem rillenförmigen kapillaren Sammelkanal versehenen flachen Stechelements.

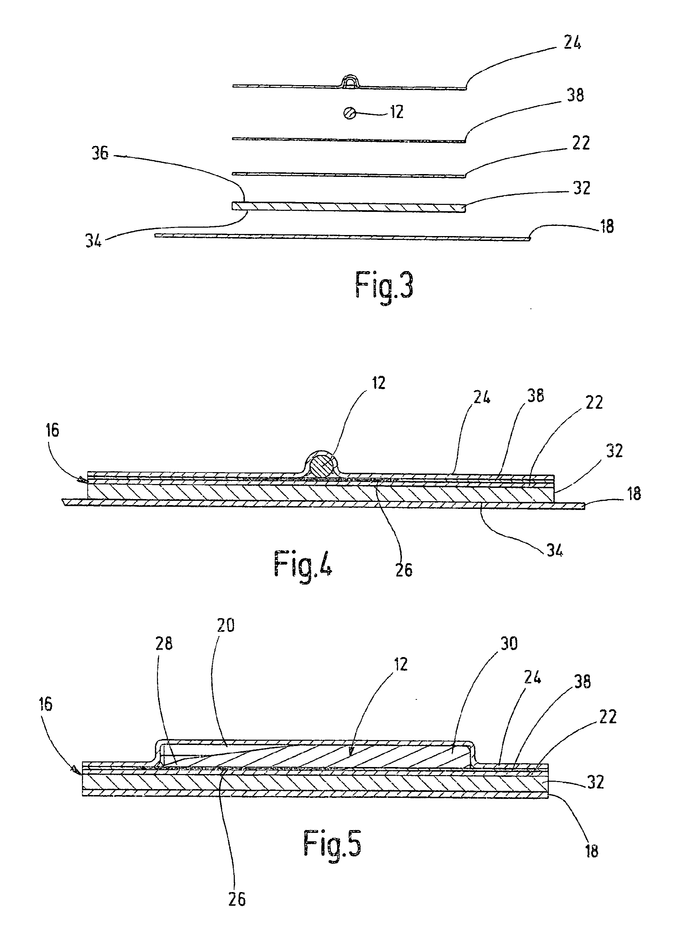

Fig. 3 zeigt die einzelnen Bestandteile des Folienaufbaus nach Fig. 2. Als Trägerband 18 kann beispielsweise eine ca. 10 µm dicke und ca. 5 - 10 mm breite PET-Folie dienen. Die Verbindung der etikettenartigen Lanzetten-Packung 16 vermittelt ein Abschnitt eines doppelseitigen Klebebands 32, welches eine erste Klebefläche 34 für das Trägerband 18 und eine gegenüberliegende zweite Klebefläche 36 für die Bodenfolie 22 besitzt. Alternativ lässt sich auch ein einseitig klebendes Klebebandstück einsetzen, das zugleich die Bodenfolie bildet. Anstelle einer Klebeverbindung ist auch eine sonstige stoffschlüssige oder mechanische Verbindung (z.B. Klettverschluss) denkbar. Der Verbund zwischen Bodenfolie 22 und Deckfolie 24 unter Einschluss der Lanzette 12 erfolgt über eine bodenfolienseitige Schmelzkleberschicht 38, die durch einen Heißstempel aktiviert wird, wie nachstehend näher erläutert.

Fig. 4 und 5 zeigen das Fertigprodukt nach Fig. 2 in einem Schnitt quer und längs zu der Lanzette 12. Die dünne Deckfolie schmiegt sich über den Lanzettenquerschnitt an, so dass eine Versiegelung rund um die Lanzette 12 erfolgt. Dabei ist auch zu ersehen, dass der proximale Lanzettenabschnitt 30 über die Schmelzkleberschicht 38 auf der Bodenfolie 22 fixiert ist, während der distale Lanzettenabschnitt 28 in dem erweiterten unverschweißten Bereich 26 der Tasche 20 für die Benutzung freigehalten wird. Vor allem in der gezeigten Querausrichtung der Lanzetten 12 quer zur Bandlängsrichtung ist es zweckmäßig, wenn die Lanzetten-Packungen 16 unter Berücksichtigung von Fertigungstoleranzen entsprechend der Breite des Trägerbandes 18 dimensioniert werden.

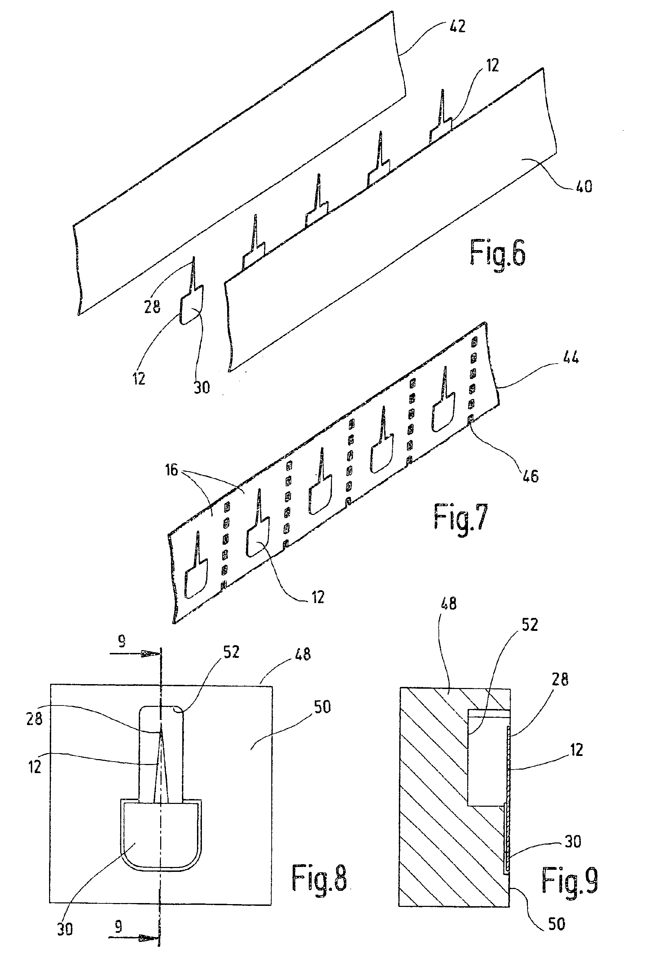

Fig. 6 veranschaulicht die Vorfertigung eines Packungsbandes mit einer Vielzahl von zusammenhängenden Lanzetten-Packungen. Zu diesem Zweck werden ein Bodenfolienstreifen 40 und ein Deckfolienstreifen 42 unter Einschluss der Lanzetten - hier Flachlanzetten 12 mit einer Lanzettenspitze 28 und einem flächigen proximalen Halterteil 30 - zusammengeführt und fest miteinander verbunden. Als Bodenfolienstreifen 40 kann beispielsweise eine etwa 20 µm dicke PET-Folie eingesetzt werden, die einseitig mit einem Heißkleber aus einem niedrigschmelzenden Polyester, z.B. einem Ethylenvinylacetat-Copolymer beschichtet ist. Der Abdeckfolienstreifen 42 ist zweckmäßig dünner als die Bodenfolienstreifen 40 ausgebildet, beispielsweise als 5 µm dicke PET-Folie.

Wie in Fig. 7 gezeigt, wird das Packungsband 44 mit quer verlaufenden Perforationen 46 versehen, die eine Vereinzelung der Lanzetten-Packungen 16 vor dem Aufbringen auf das Trägerband 18 vereinfachen. Möglich ist es auch, die einzelnen Packungen durch heiße Schneidmittel zugleich zu zerteilen und entlang der Schnittlinie zu verschweißen, wobei thermoplastisches Folienmaterial eingesetzt wird. Eine weitere Variante sieht vor, dass ein längs gefalteter Folienstreifen mit Lanzetten bestückt und quer in einzelne Packungen zerteilt wird (nicht gezeigt). Hierbei bildet eine Längshälfte des Folienstreifens den Bodenbereich zum Aufbringen der Lanzetten, während die andere Längshälfte anschließend übergefaltet und mit dem Bodenbereich verbunden wird.

Fig. 8 und 9 zeigen die Versiegelung der einzelnen Lanzetten-Packungen durch einen auf den Schmelzkleber einwirkenden Heißstempel 48. Dieser ist an seiner auf die jeweilige Lanzetten-Packung 16 angedrückten Stempelfläche 50 mit einer abgestuften Aussparung 52 versehen, welche den proximalen Nadelabschnitt 30 eng umschließt und durch eine entsprechende Vertiefung für den erweiterten unverschweißten Bereich 26 der Tasche 20 im Bereich der Nadelspitze 28 sorgt. Hierbei wird der proximale Nadelabschnitt 30 bevorzugt mit der Bodenfolie 22 fest verklebt, so dass die Nadel 12 nach einer späteren Verwendung in Verbindung mit dem Trägerband 18 remagaziniert werden kann.

Wie nicht eigens dargestellt, können die in den Lanzetten-Packungen 16 eingeschweißten Lanzetten 12 durch Bestrahlung beispielsweise mit einem hochenergetischen Elektronenstrahl sterilisiert werden. Aufgrund der stoffdichten Versiegelung ist die Sterilhaltung auch im weiteren Verarbeitungsablauf gewährleistet.

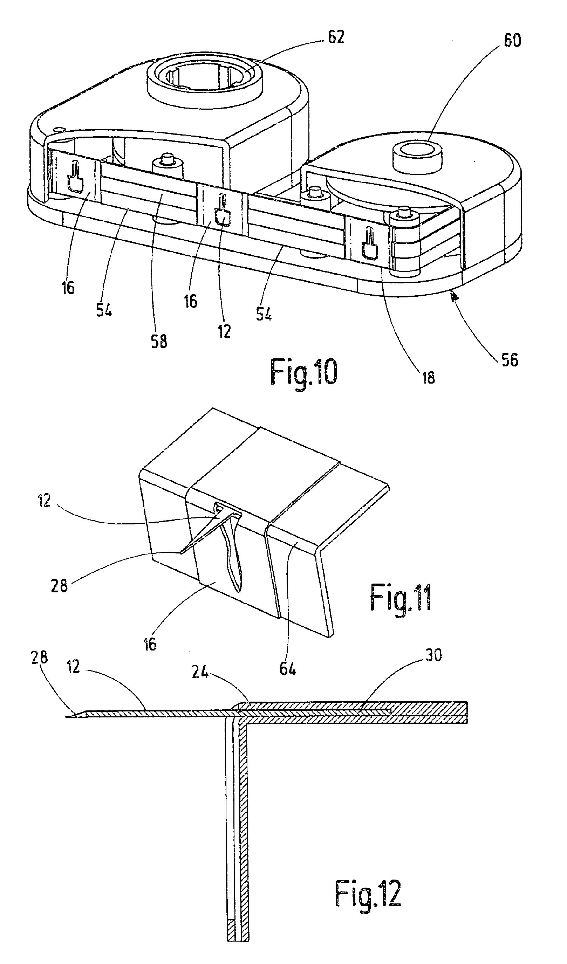

Die Lanzetten-Packungen 16 werden an einer Etikettierstation auf das Trägerband 18 aufetikettiert. Hierbei können auch zusätzlich Testelemente in Form von Testfeld-Etiketten 54 aufgebracht werden, wie es in Fig. 10 am Beispiel einer Bandkassette 56 ersichtlich ist. Die Testfeld-Etiketten 54 sind mit einer Reagenzschicht 58 versehen, die mit durch einen Hauteinstich gewonnener Körperflüssigkeit (Blut und/oder Gewebeflüssigkeit) beaufschlagbar ist, um einen Analyten (Glucose) nachzuweisen. Der Nachweis erfolgt vorzugsweise durch eine Farbänderung, kann aber auch elektrochemischer Natur sein. Die maschinelle Handhabung der unterschiedlichen disposiblen Einheiten 16, 54 an der Etikettierstation kann somit in einem einheitlichen Etikettier-Prozess erfolgen, wie er nur für Testfeld-Etiketten in der EP-A 1 593 434 beschrieben ist, worauf hier Bezug genommen wird.

Die Kassette 56 besitzt eine Vorratsspule 60 für unverbrauchtes Bandmaterial und eine Aufwickelspule 62 zur Remagazinierung bzw. Entsorgung von verbrauchten Einheiten 16, 54. Die Bereitstellung erfolgt durch sukzessiven Bandvorschub vorzugsweise in einem Handgerät, um einen weitgehend automatischen Messablauf zu ermöglichen.

Im Zuge einer solchen Messung, die vom Patienten vor Ort selbst vorgenommen werden kann, wird die jeweilige Lanzette 12 gemäß Fig. 11 und 12 zum Einsatz gebracht. Durch Knicken der Lanzetten-Packung 16 an einer Umlenkstelle 64 wird die dünne Deckfolie 24 von der Lanzette 12 aufgeschlitzt und dabei die Lanzettenspitze 28 freigelegt. Durch einen geeigneten Aktuator kann dann eine Stechbewegung beispielsweise zum Einstechen in einen Finger durchgeführt werden. Der proximale Lanzettenabschnitt 30 bleibt hierbei zweckmäßig mit dem Folienlaminat verbunden, so dass die anschließende Entsorgung der Lanzette 12 auf dem Trägerband 18 vereinfacht wird.

## Patentansprüche

1. Verfahren zur Magazinierung von Lanzetten insbesondere für Blutglucoseuntersuchungen bei dem eine Vielzahl von mit einer Lanzettenspitze (28) zum Einstechen in die Haut versehenen disposiblen Lanzetten (12) auf einem auf einer Spule (60,62) aufgewickelten oder einem aufwickelbaren Trägerband (18) zum Gewinnen einer Körperflüssigkeitsprobe bereitgestellt werden, wobei die Lanzetten in Bandquerrichtung des Trägerbandes (18) ausgerichtet sind, **dadurch gekennzeichnet, dass** die Lanzetten (12) in jeweils einer Packung (14) angeordnet und dabei zumindest im Bereich ihrer Spitze vor dem Aufbringen auf das Trägerband (18) sterilisiert und steril eingeschlossen werden, und dass anschließend die so gebildeten Lanzetten-Packungen (16) einzeln auf das Trägerband (18) aufgebracht und darauf fixiert werden.

2. Verfahren nach Anspruch 1, **dadurch gekennzeichnet, dass** die Packungen (14) aus einer Bodenfolie (22) und einer damit verbundenen Deckfolie (24) gebildet werden.

3. Verfahren nach Anspruch 2, **dadurch gekennzeichnet, dass** die Bodenfolie (22) und die Deckfolie (24) unter Einschluss der Lanzetten (12) miteinander in Kontakt gebracht und verbunden werden.

4. Verfahren nach einem der Ansprüche 1 bis 3, **dadurch gekennzeichnet, dass** eine Vielzahl von Lanzetten-Packungen (16) als Packungsband (44) zusammenhängend gefertigt und vor dem Aufbringen auf das Trägerband (18) vereinzelt wird.

5. Verfahren nach einem der Ansprüche 1 bis 4, **dadurch gekennzeichnet, dass** die Lanzetten-Packungen (16) durch einen Heißschnitt zugleich zugeschnitten und unter Einschluss der Lanzetten (12) dicht umrandet werden.

6. Verfahren nach einem der Ansprüche 1 bis 5, **dadurch gekennzeichnet, dass** die Lanzetten (12) vorzugsweise an einem proximalen Endabschnitt (30) fest mit der Packung (14) verbunden werden.

7. Verfahren nach einem der Ansprüche 1 bis 6, **dadurch gekennzeichnet, dass** die Lanzettenspitzen (28) in einer durch die Packung (14) gebildeten Tasche (20) angeordnet werden.

8. Verfahren nach Anspruch 1, **dadurch gekennzeichnet, dass** die Lanzetten-Packungen (16) durch eine Bodenfolie (22) und eine darauf aufgebrachte und zumindest die Lanzettenspitze (28) umschließende, insbesondere aus Silikon bestehende Verschlussmasse gebildet werden.

9. Verfahren nach einem der Ansprüche 1 bis 8, **dadurch gekennzeichnet, dass** zumindest die Spitzen (28) der Lanzetten (12) in den Lanzetten-Packungen (16) vor dem Aufbringen auf das Trägerband (18) durch hochenergetische Bestrahlung sterilisiert werden.

10. Verfahren nach einem der Ansprüche 1 bis 9, **dadurch gekennzeichnet, dass** die Lanzetten-Packungen (16) in Form von Etiketten oder Aufklebern auf das Trägerband (18) aufgebracht werden.

11. Verfahren nach einem der Ansprüche 1 bis 10, **dadurch gekennzeichnet, dass** die Lanzetten-Packungen (16) durch mechanische Verbindungsmittel oder durch Verschweißen auf dem Trägerband (18) gehalten werden.

12. Verfahren nach einem der Ansprüche 1 bis 11, **dadurch gekennzeichnet, dass** auf dem Trägerband (18) vorzugsweise alternierend zu den Lanzetten-Packungen (16) mit der Köperflüssigkeitsprobe beaufschlagbare Testelemente (54) aufgebracht werden.

13. Verfahren nach Anspruch 12, **dadurch gekennzeichnet, dass** die Lanzetten (12) und die Testelemente (54) gesondert als Packungseinheiten vorgefertigt werden, und dass anschließend in einem einheitlichen Bestückungsprozess die Packungseinheiten auf das Trägerband (18) aufgebracht und darauf fixiert werden.

14. Verfahren nach Anspruch 12 oder 13, **dadurch gekennzeichnet, dass** die flachen Lanzetten- und Testelement-Packungseinheiten eine im Wesentlichen übereinstimmende, vorzugsweise der Breite des Trägerbands (18) entsprechende Breite aufweisen.

15. Verfahren nach einem der Ansprüche 1 bis 14, **dadurch gekennzeichnet, dass** das Trägerband (18) in einer Kassette (56) angeordnet wird, so dass es von einer Abwickelspule (60) abziehbar und auf eine Aufwickelspule (62) aufspulbar ist.

## Claims

1. Method for storing lancets in particular for blood glucose analyses in which a plurality of disposable lancets (12) provided with a lancet tip (28) for piercing the skin are provided on a carrier tape (18) which is wound onto or can be wound onto a spool (60, 62) for obtaining a sample of body fluid, wherein the lancets (12) are aligned in tape transverse direction of the carrier tape (18), **characterized in that** the lancets (12) are arranged each in a package (14) and are sterilized and thereby sterilely enclosed at least in the area of their tip before they are applied to the carrier tape (18), and that subsequently the lancet packages (16) formed in this manner are applied individually to the carrier tape (18) and attached thereon.

2. Method according to claim 1, **characterized in that** the packages (14) are formed from a base foil (22) and a cover foil (24) that is joined thereto.

3. Method according to claim 2, **characterized in that** the base foil (22) and cover foil (24) are brought into contact with one another and joined while enclosing the lancets (12).

4. Method according to one of the claims 1 to 3, **characterized in that** a plurality of lancet packages (16) are manufactured in a connected manner as a package tape (44) and are separated before application to the carrier tape (18).

5. Method according to one of the claims 1 to 4, **characterized in that** the lancet packages (16) are at the same time cut and the border is sealed while enclosing the lancets (12) by means of a hot cut.

6. Method according to one of the claims 1 to 5, **characterized in that** the lancets (12) are permanently joined to the package (14) preferably at a proximal end section (30) of the said lancets.

7. Method according to one of the claims 1 to 6, **characterized in that** the lancet tips (28) are arranged in a pocket (20) formed by the package (14).

8. Method according to claim 1, **characterized in that** the lancet packages (16) are formed by a base foil (22) and a sealing compound applied thereon especially consisting of silicone which encloses at least the lancet tip (28).

9. Method according to one of the claims 1 to 8, **characterized in that** at least the tips (28) of the lancets (12) in the lancet packages (16) are sterilized by high-energy irradiation before they are applied to the carrier tape (18).

10. Method according to one of the claims 1 to 9, **characterized in that** the lancet packages (16) are applied to the carrier tape (18) in the form of labels or stickers.

11. Method according to one of the claims 1 to 10, **characterized in that** the lancet packages (16) are held on the carrier tape (18) by mechanical connecting means or by welding.

12. Method according to one of the claims 1 to 11, **characterized in that** test elements (54) to which a sample of body fluid can be applied are applied to the carrier tape (18) preferably alternating with the lancet packages (16).

13. Method according to claim 12, **characterized in that** the lancets (12) and the test elements (54) are prefabricated separately as package units and that subsequently the package units are applied to and fastened on the carrier tape (18) in a common assembly process.

14. Method according to claim 12 or 13, **characterized in that** the flat lancet and test element package units have an essentially corresponding width which preferably corresponds to the width of the carrier tape (18).

15. Method according to one of the claims 1 to 14, **characterized in that** the carrier tape (18) is arranged in a cassette (56) so that it can be pulled off a take-off spool (60) and wound onto a take-up spool (62).

## Revendications

1. Procédé de stockage de lancettes destinées notamment à l'analyse de la glycémie, consistant à mettre à disposition une pluralité de lancettes (12) à usage unique, lesquelles sont pourvues d'une pointe de lancette (28) destinée à pénétrer dans la peau, sur un ruban de support (18) enroulé sur une bobine (60, 62) ou pouvant être enroulé, pour ainsi prélever un échantillon d'un liquide corporel, lesdites lancettes étant orientées dans une direction transversale au ruban de support (18), **caractérisé en ce que** les lancettes (12) sont chacune disposées dans un emballage (14) pour ainsi être stérilisées et enfermées dans un espace stérile, au moins en ce qui concerne la zone entourant leur pointe, avant d'être appliquées sur le ruban de support (18) et que les emballages à lancette (16) ainsi formés sont appliqués un à un sur le ruban de support (18) et fixés sur ce dernier.

2. Procédé selon la revendication 1, **caractérisé en ce que** les emballages (14) sont formés à partir d'une feuille de fond (22) et d'une feuille de couverture (24) reliée à cette dernière.

3. Procédé selon la revendication 2, **caractérisé en ce que** la feuille de fond (22) et la feuille de couverture (24) sont mises en contact et reliées l'une à l'autre en enfermant les lancettes (12).

4. Procédé selon l'une des revendications 1 à 3, **caractérisé en ce qu'**une pluralité d'emballages à lancette (16) sont fabriqués sous forme d'un ruban d'emballage (44) constitué d'emballages contigus lesquels sont isolés les uns des autres avant d'être appliqués sur le ruban de support (18).

5. Procédé selon l'une des revendications 1 à 4, **caractérisé en ce que** les emballages à lancette (16) sont découpés en réalisant une découpe à chaud laquelle provoque en même temps un scellement en bordure enfermant les lancettes (12).

6. Procédé selon l'une des revendications 1 à 5, **caractérisé en ce que** les lancettes (12) sont de préférence solidaires avec une partie (30) de l'emballage (14) située à l'extrémité proximale.

7. Procédé selon l'une des revendications 1 à 6, **caractérisé en ce que** les pointes de lancette (28) sont disposées dans une poche (20) formée par l'emballage (14).

8. Procédé selon la revendication 1, **caractérisé en ce que** les emballages à lancette (16) sont formés par une feuille de fond (22) et un matériau de scellement constitué notamment de silicone lequel est appliqué sur cette dernière et lequel entoure au moins la pointe de lancette (28).

9. Procédé selon l'une des revendications 1 à 8, **caractérisé en ce qu'**au préalable de l'application sur le ruban de support (18), au moins les pointes (28) des lancettes (12) sont stérilisées dans les emballages à lancette (16) au moyen d'un rayonnement à haute énergie.

10. Procédé selon l'une des revendications 1 à 9, **caractérisé en ce que** les emballages à lancette (16) sont appliqués sur le ruban de support (18) sous forme d'étiquettes ou d'autocollants.

11. Procédé selon l'une des revendications 1 à 10, **caractérisé en ce que** les emballages à lancette (16) sont maintenus sur le ruban de support (18) à l'aide de moyens de liaison mécaniques ou par soudage.

12. Procédé selon l'une des revendications 1 à 11, **caractérisé en ce que** des éléments d'essai (54) pouvant être exposés audit échantillon de liquide corporel sont appliqués sur la bande de support (18), de préférence en
alternance avec les emballages à lancette (16).

13. Procédé selon la revendication 12, **caractérisé en ce que** les lancettes (12) et les éléments d'essai (54) sont préfabriqués sous forme d'unités d'emballage et que lesdites unités d'emballage sont ensuite appliquées et fixées sur le ruban de support (18) selon un processus d'équipement uniforme.

14. Procédé selon les revendications 12 ou 13, **caractérisé en ce que** les unités d'emballage à lancette et à élément d'essai sont plates et présentent une largeur sensiblement identique correspondant de préférence à la largeur du ruban de support (18).

15. Procédé selon l'une des revendications 1 à 14, **caractérisé en ce que** le ruban de support (18) est disposé dans une cassette (56) de manière à ce qu'il puisse être déroulé depuis une bobine de déroulement (60) et enroulé sur une bobine d'enroulement (62).
